Europäisches Patentamt

European Patent Office

Office européen des brevets

(19)

(11) Veröffentlichungsnummer: 0 159 522
A1

## (12) EUROPÄISCHE PATENTANMELDUNG

(21) Anmeldenummer: 85102954.6

(22) Anmeldetag: 14.03.85

(51) Int. Cl.⁴: **A 61 K 31/48**
// C07D457/12

(30) Priorität: 16.03.84 DE 3410218

(43) Veröffentlichungstag der Anmeldung: 30.10.85
Patentblatt 85/44

(84) Benannte Vertragsstaaten: **AT BE CH DE FR GB IT LI LU
NL SE**

(71) Anmelder: **SCHERING AKTIENGESELLSCHAFT Berlin
und Bergkamen,
Müllerstrasse 170/178 Postfach 65 03 11,
D-1000 Berlin 65 (DE)**

(72) Erfinder: **Kehr, Wolfgang, Dr., Biedermannweg 11,
D-1000 Berlin 19 (DE)**
Erfinder: **Schröder, Gertrud, Dr.,
Theodor-Franke-Strasse 3, D-1000 Berlin 42 (DE)**
Erfinder: **Stock, Günter, Prof., Ostpreussendamm 152,
D-1000 Berlin 45 (DE)**
Erfinder: **Wachtel, Helmut, Dr., Suarezstrasse 22,
D-1000 Berlin 19 (DE)**

(54) 3-(6-Methylergolin-8 alpha-yl)-1.1-diethyl-Harnstoff als Antihypertensivum.

(57) Es wird die neue Verwendung von 3-(6-Methylergolin-8α-yl)-1.1-diethylharnstoff und dessen physiologisch verträglichen Salzen in einer Dosis von 0,1–1,0 mg/Tag zur Behandlung der essentiellen Hypertonie in der Humanmedizin beschrieben.

EP 0 159 522 A1

BEZEICHNUNG GEÄNDERT
Siehe Titelseite

Die Erfindung betrifft den Gegenstand der Patentansprüche.

Tergurid (3-(6-Methylergolin-8α-yl)-1.1-diethylharnstoff)
selbst und dessen nidations- und laktationshemmende sowie dessen antipsychotische Wirkung bei oraler Anwendung
am Tier und am Menschen aufgrund seiner partialagonistischen Wirkung an Dopaminrezeptoren sind bekannt
(DE PS 22 38 540, DE OS 31 29 714).

Als physiologisch verträgliche Salze kommen Salze von
Tergurid mit anorganischen und organischen Säuren in
Frage. Zur Salzbildung geeignet sind zum Beispiel Salzsäure, Phosphorsäure, Schwefelsäure, Methansulfonsäure,
Glucoheptansäure, Bernsteinsäure, Weinsäure, Maleinsäure
usw. Ein bevorzugtes Salz ist das Tergurid-dihydrogenphosphat.

Es ist bekannt, daß Dopaminagonisten einerseits über eine
direkte Dilatation der Gefäße und andererseits aufgrund
einer verminderten Noradrenalinausschüttung aus sympathischen
Nervenendigungen zu einer Blutdrucksenkung führen.
.(Cavero, I. et al., Life Science, 31, 939-948 und
1059-1069 (1982).

Aus diesem Grund sind auch bereits Dopaminagonisten wie
verschiedene Mutterkornalkaloide, z.B. Bromocriptin oder
Lisurid, in der Humanmedizin zur Hochdrucktherapie verwendet worden (Stumpe, K.O., Kolloch, R., Higuchi, M.K.,
Kruck, F., Vetter, H.: Hyperprolactinemia and antihypertensive effect of bromocryptine in essential hypertension,
Lancet 2:211, 1977).

Diese Verbindungen haben jedoch den Nachteil, daß wegen der gleichzeitigen Stimulation der Dopamin-Rezeptoren in der Area postrema mit Erbrechen als unerwünschte Nebenwirkung zu rechnen ist.

Aufgabe der vorliegenden Erfindung war es, ein blutdrucksenkendes Arzneimittel bereitzustellen, das diese Nachteile überwindet.

Es wurde nun gefunden, daß Tergurid antihypertensiv wirkt, ohne eine emetische Nebenwirkung auszulösen.

In tierpharmakologischen Untersuchungen zeigte Tergurid eine dosisabhängige Blutdrucksenkung an spontan-hypertensiven Ratten, die nach einer Methode modifiziert nach Weeks präpariert wurden (Weeks, J.R., Routine Direct Measurement of Arterial Pressure in Unaesthetized Rats. Proc.S.Exp.Biol.Med. 104: 646-648, 1960).

Zur Untersuchung der blutdrucksenkenden Wirkung und ihrer Dosisabhängigkeit wurden folgende pharmakologische Teste ausgeführt:

Bei ca. 300 g schweren, männlichen SH-Ratten wurde mittels eines implantierten Aortenkatheters der mittlere arterielle, der systolische und der diastolische Blutdruck bestimmt. Tergurid in einer Dosierung von 200 µg/kg Körpermasse (KM) wurde über einen in der Vena jugularis plazierten Katheter im Bolus verabreicht.

Der Dopaminantagonist Haloperidol (1 mg/kg i.p.) wurde 45 Minuten vor der intravenösen Injektion von Tergurid verabreicht.

Tergurid verursacht in der verwendeten Dosis eine biphasische Blutdrucksenkung, die größtenteils durch Haloperidolvorgabe antagonisierbar ist.

Männliche SH-Ratten, die wie oben angegeben präpariert wurden, erhielten jeweils 10, 75, 150 und 225 µg/kg KM Tergurid. Tergurid bewirkt eine dosisabhängige Blutdrucksenkung, deren Maximum bei 150 µg/kg KM bereits erreicht ist. Durch Erhöhung der Dosis auf 225 µg/kg KM ist nur noch eine marginale Verlängerung der deutlich antihypertensiven Phase feststellbar.

Das Maximum der Blutdrucksenkung beträgt ca. 28-30 % des Ausgangswertes. Dieses Niveau ist ca. 20 Minuten nach der Applikation erreicht.

Die Herzfrequenz wird durch Tergurid dosisabhängig erhöht. Diese Erhöhung ist bereits 13 Minuten nach Applikation abgeklungen und liegt zeitlich vor dem maximalen Blutdruckabfall.

An normotensiven, männlichen, ca. 300 g schweren Ratten wurde des weiteren die Wirkung von Tergurid auf die Kardio- und Hämodynamik untersucht.

Die Tiere wurden mit 70 bis 100 mg/kg KM Na-Phentobarbital i.p. narkotisiert. Zur Blutdruckmessung wurde ein Katheter via A. femoralis in die Bauchaorta vorgeschoben. Der Injektionskatheter für die Prüfsubstanz wurde in der rechten V. femoralis plaziert. Über einen Katheter im linken Ventrikel konnte der Ventrikeldruck abgenommen werden und daraus $dp/dt_{max}$ abgeleitet werden. Das Herzminutenvolumen wurde mit der Kälteverdünnungsmethode bestimmt. Aus EKG-Aufzeichnung (Ableitung in der Herzachse) wurde die Herzfrequenz bestimmt.

150 µg/kg KM Tergurid verursachen an narkotisierten Tieren einen kurzfristigen, ca. 5 Minuten anhaltenden Abfall des mittleren arteriellen Blutdruckes. Die maximale Senkung beträgt ungefähr 40 % des Ausgangswertes.

Der periphere Widerstand nimmt initial um ca. 40 % des Ausgangswertes ab und ist nach 5 Minuten bereits wieder auf 75 % des Ausgangswertes angestiegen.

Die Herzfrequenz wird nicht beeinflußt. Zeitgleich mit dem Blutdruck fällt $dp/dt_{max}$ ab, was durch eine blutdruckbedingte Afterloadreduktion zu erklären ist.

Weiterhin wurde die $\alpha_1$-adrenolytische und direkt vasodilatierende Wirkung von Tergurid an isolierten, spiralig geschnittenen Aortenstreifen der Ratte durch die Beeinflussung einer mit $10^{-7}$ Mol Noradrenalin/l Badflüssigkeit induzierten Kontraktion von $10^{-10}$ bis $3 \times 10^{-5}$ Mol/l Tergurid nach der Methode von Furchgott (Furchgott, R.F., Bhadrakon S., Reactions of strips of rabbit aorta to epinephrine, isopropylarterenol, sodium nitrite and other drugs, J.Pharmacol. 108: 129-143, 1953) untersucht.

Die $\alpha_1$-andrenolytische Wirkungsstärke (Bestimmung des $pA_2$-Wertes) wurde nach der Methode von Arunlakshana und Schild (Arunlakshana, O., Schild, H.O.; Some quantitative uses of drug antagonists, Brit.J.Pharmacol. 14: 48-58 (1959)) ermittelt.

An $K^+$-depolarisierten Aortenstreifen der Ratte wurde eine direkt vasodilatatierende Einschaft untersucht.

Tergurid verschiebt die Noradrenalin-Dosiswirkungskurve parallel nach rechts, so daß der $pA_2$-Wert für Tergurid 7.99 beträgt. Eine Beeinflussung der Kontraktion an $K^+$-depolarisierten Aortenstreifen nach Zugabe von Tergurid war jedoch nicht feststellbar.

Aus den Ergebnissen der tierexperimentellen kreislaufpharmakologischen Untersuchungen wird gefolgert, daß mit Tergurid über dopamin-agonistische Wirkung eine Blutdrucksenkung erzielt wird. Die $\alpha$-adrenolytischen Effekte tragen dagegen nur unwesentlich zur Blutdrucksenkung bei.

Die dopamin-agonistische Wirkqualität wurde in einer humanpharmakologischen Cross-over-Studie an 15 normotonen Versuchspersonen bestätigt, wobei als Parameter für die dopamin-agonistische Wirkung u.a. die Prolaktinsenkung herangezogen wurde. Die Plasmaprolaktinspiegel wurden nach einmaliger oraler Gabe von 0,2, 0,5 und 1,0 mg Tergurid bis 24 Stunden p.appl. dosisabhängig gesenkt. Lediglich nach 1 mg Tergurid wurde bei 2 von 15 normotonen Versuchspersonen ein orthostatischer Blutdruckabfall und eine leichte Nausea beobachtet. Ansonsten wurden der Blutdruck und die Herzfrequenz dieser Versuchspersonen nicht beeinträchtigt.

In Analogie zu den tierexperimentellen Befunden ist davon auszugehen, daß Tergurid ein Arzneimittel ist, das aufgrund der dopamin-agonistischen Wirkung eine Blutdrucksenkung an hypertensiven, nicht jedoch an normotensiven Patienten herbeiführt, wobei die Gefahr einer gehäuften Emesisauslösung im blutdrucksenkenden Bereich nicht zu erwarten ist.

In der medizinischen Praxis können Arzneimittel auf Basis von Tergurid subcutan, intramuskulär, intravenös und vor allem per os appliziert werden. Die tägliche Dosis beträgt 0,1 - 1,0 mg.

Die Herstellung der Arzneimittelspezialitäten erfolgt in an sich bekannter Weise, indem das Tergurid mit den in der Pharmazie gebräuchlichen Trägersubstanzen, Verdünnungsmitteln, Geschmackskorrigentien usw. verarbeitet wird. Für Injektionen kommen insbesondere wässrige, aber auch ölige Lösungen sowie Suspensionen in Frage. Zur Herstellung intramuskulärer Depotformen können die Wirkstoffe nach gängigen Methoden in fetten Ölen suspendiert oder gelöst werden.

Die erfindungsgemäßen Arzneimittel sind insbesondere in Form von Tabletten, Kapseln, Dragees, Pillen, Suspensionen und Lösungen für die orale Applikation geeignet. Geeignet sind aber auch orale Retardformen, die in üblicher Weise, z.B. durch Zugabe von hydrierten Fetten und Verarbeitung mit Harzbildnern und Lacken, erhalten werden. Tropfen
für die orale Applikation können durch wässrige Lösungen
oder Suspensionen des Wirkstoffes in Ölen unter Zugabe
von Geschmackskorrigenten und/oder Lösungsvermittlern
hergestellt werden.

0159522

## Patentansprüche

1. Verwendung von Tergurid und dessen physiologisch verträglichen Salzen zur Behandlung der essentiellen Hypertonie.

2. Verwendung nach Anspruch 1, dadurch gekennzeichnet, daß man Tergurid in der Humanmedizin in einer Dosis von 0,1 - 1,0 mg/Tag appliziert.

3. Verwendung nach Anspruch 1, dadurch gekennzeichnet, daß man Tergurid allein oder mit anderen galenischen Hilfsstoffen formuliert anwendet.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 056 358 (SCHERING AG) <br> * Ansprüche * <br> --- | 1 | A 61 K 31/48 // <br> C 07 D 457/12 |
| A | FR-A-2 421 176 (SANDOZ S.A.) <br> * Seite 4; Ansprüche * <br> --- | 1 | |
| D,A | DE-A-3 129 714 (SCHERING AG) <br> * Anspruch * <br> --- | | |
| D,A | DE-B-2 238 540 (SPOFA) <br> * Spalten 1,2 * <br> ----- | 1 | |
| | | | **RECHERCHIERTE SACHGEBIETE (Int. Cl.4)** |
| | | | C 07 D 457/00 |

Der vorliegende Recherchenbericht wurde für alle Patentansprüche erstellt.

| Recherchenort <br> DEN HAAG | Abschlußdatum der Recherche <br> 20-06-1985 | Prüfer <br> VAN BIJLEN H. |
|---|---|---|